# FASCICULE DE BREVET EUROPEEN

(11) **EP 2 788 367 B1**
(45) Date de publication et mention de la délivrance du brevet: **09.03.2016**
(21) Numéro de dépôt: 12810354.6
(22) Date de dépôt: 05.12.2012
(51) Int. Cl.: C07F 15/00, A61K 41/00

(54) **APPLICATION MÉDICALE OU THÉRAPEUTIQUE D'UN MATÉRIAU COMPOSITE DE RUTHÉNIUM À LIGAND NITROSYLE**
MEDIZINISCHE ODER THERAPEUTISCHE ANWENDUNG EINES VERBUNDWERKSTOFFS AUS RUTHENIUM MIT EINEM NITROSYLLIGANDEN
MEDICAL OR THERAPEUTIC APPLICATION OF A COMPOSITE MATERIAL OF RUTHENIUM WITH A NITROSYL LIGAND

(30) Priorité: 06.12.2011 FR 1161223
(43) Date de publication de la demande: 15.10.2014
(73) Titulaire: Centre National de la Recherche Scientifique (CNRS), 75016 Paris (FR)
(72) Inventeur: MALFANT, Isabelle, F-31120 Goyrans (FR); CORMARY, Benoit, F-31450 Montgiscard (FR)
(74) Mandataire: Derambure Conseil
(86) Numéro de dépôt international: PCT/FR2012/052815
(87) Numéro de publication internationale: WO 2013/083921

(56) Documents cités:
- WO-A1-2010/081977
- CORMARY B. ET AL.: "New Photochromic Xerogels Composites Based on Nitrosyl Complexes", J SOL-GEL TECHNOL, vol. 52, 1 janvier 2009 (2009-01-01), pages 19-23, XP002679200,
- SCHANIEL D. ET AL.: "Photogeneration of two metastable NO linkage isomers with high populations of up to 76% in trans-[RuCl(py)4(NO)][PF6]2.1/2H2O", PHYSICAL CHEMISTRY CHEMICAL PHYSICS, vol. 9, 30 mai 2007 (2007-05-30), pages 3717-3724, XP002679201,

## Description

L'invention est relative à l'application pour un traitement médical ou thérapeutique d'un matériau composite de ruthénium à ligand nitrosyle.

L'on connaît déjà les applications thérapeutiques de la molécule NO concernant nombre de processus physiologiques, tels que la neurotransmission et la destruction de cellules cancéreuses. C'est ainsi que Robert F. Furchgott, Ferid Murad et Louis J. Ignarro se sont vu attribuer le prix Nobel de Médecine et de Physiologie 1988 pour avoir établi le rôle décisif de NO dans la physiologie cardiovasculaire. Et l'on sait que si NO a un rôle anti apoptotique, il a également un rôle pro apoptotique.

L'invention vise à permettre de tirer un meilleur parti du rôle de NO dans le domaine médical ou thérapeutique.

Le document WO 2010/081977 décrit un procédé sol-gel de production d'un matériau composite photochrome, un tel matériau composite photochrome ainsi réalisé, et l'application d'un tel matériau comme média à mémoire optique de haute qualité, notamment capacité.

L'invention repose sur le constat surprenant qu'un tel matériau ou un matériau dérivé pouvait également être appliqué pour un traitement médical ou thérapeutique fondé sur le rôle de NO.

L'invention a donc pour objet un matériau composite de ruthénium à ligand nitrosyle propre à libérer le ligand nitrosyle lorsqu'il est irradié dans une gamme spectrale, pour son utilisation dans un traitement médical ou thérapeutique, du corps humain ou animal.

Comme décrit plus en détail ci-dessous, ledit matériau composite de ruthénium à ligand nitrosyle comprend un complexe photochrome de ruthénium à ligand nitrosyle de formule :
o [Ru(NO)X(py-R)₄]Y₂, [Ru(NO)X(py-RR')₄]Y₂, [Ru(NO)X(py-RR'R")₄]Y₂ ou [Ru(NO)X(py)₄]Y₂ où :
   - py désigne la pyridine et Ru le ruthénium,
   - X est choisi dans la famille comprenant Cl, Br, OH,
   - Y est choisi dans la famille comprenant Cl, Br, BF₄, PF₆,
   - R, R', R" est choisi dans la famille comprenant l'hydrogène, les groupements alkyle, les groupements alcool, les groupements aldéhyde, les groupements cétone, les groupements ester, les groupements éther, les groupements amine, les groupements amide et les groupements halogénés,
o ou de formule [RuCl(NO)(py)4](PF6]2.½H₂O.

Selon une mise en oeuvre, le matériau composite de ruthénium à ligand nitrosyle est propre à libérer le ligand nitrosyle de façon locale et photocontrôlée lorsqu'il est irradié dans une gamme spectrale, telle que la gamme spectrale de l'ultraviolet et du visible ou celle du proche infrarouge.

Selon une mise en oeuvre, le matériau composite de ruthénium à ligand nitrosyle est en solution.

Selon une mise en oeuvre possible, le matériau composite de ruthénium à ligand nitrosyle est irradié dans la gamme spectrale de l'ultraviolet et du visible, c'est-à- dire dans une gamme de longueur d'ondes comprise entre 300 nm et 700 nm.

Selon une autre mise en oeuvre possible, le matériau composite de ruthénium à ligand nitrosyle est irradié dans la gamme spectrale du proche infrarouge, c'est-à-dire dans une gamme de longueur d'ondes comprise entre 700 nm et 1100 nm.

Selon une première mise en oeuvre possible, le matériau composite de ruthénium à ligand nitrosyle est réalisé par un procédé sol-gel avec :
▪ comme précurseur un alkoxysilane,
▪ une matrice de silice, et
▪ un complexe photochrome de ruthénium à ligand nitrosyle un complexe de formule :
   o [Ru(NO)X(py-R)₄]Y₂, [Ru(NO)X(py-RR')₄]Y₂, [Ru(NO)X(py-RR'R")₄]Y₂ ou [Ru(NO)X(py)₄]Y₂ où :
      - py désigne la pyridine et Ru le ruthénium,
      - X est choisi dans la famille comprenant CI, Br, OH,
      - Y est choisi dans la famille comprenant CI, Br, BF₄, PF₆,
      - R, R', R" est choisi dans la famille comprenant l'hydrogène, les groupements alkyle, les groupements alcool, les groupements aldéhyde, les groupements cétone, les groupements ester, les groupements éther, les groupements amine, les groupements amide et les groupements halogénés,
   o ou de formule [RuCl(NO)(py)₄](PF₆)₂.½H₂O.

Selon une mise en oeuvre, l'alkoxysilane est sélectionné dans le groupe comprenant le tétraméthoxyorthosilane - TMOS -, le vinyltriéthoxysilane - VTES -, le tétraéthoxyorthosilane - TEOS -, ou des alkoxydes M(OR)x, où M est un métal et R un groupement alkyle.

Selon une seconde mise en oeuvre possible, le matériau composite de ruthénium à ligand nitrosyle est réalisé avec une matrice biocompatible, en particulier d'un polysaccharide tel que l'amidon (glucane) ou l'agar-agar (galactane), et l'on sélectionne comme complexe photochrome de ruthénium à ligand nitrosyle un complexe de formule [Ru(NO)X(py-R)₄]Y₂, [Ru(NO)X(py-RR')₄]Y₂, [Ru(NO)X(py-RR'R")₄]Y₂ ou [Ru(NO)X(py)₄]Y₂ où :
- py désigne la pyridine et Ru le ruthénium,
- X est choisi dans la famille comprenant CI, Br, OH,
- Y est choisi dans la famille comprenant CI, Br, BF₄,
- R, R', R" est choisi dans la famille comprenant l'hydrogène, les groupements alkyle, les groupements alcool, les groupements aldéhyde, les groupements cétone, les groupements ester, les groupements éther, les groupements amine, les groupements amide et les groupements halogénés.

Par « biocompatible », l'on entend qui peut être reçu par le corps humain sans dommage. Des matrices de polysaccharide tel que l'amidon (glucane) ou l'agar-agar (galactane) présentent l'avantage d'être en outre biodégradable.

Un matériau tel que celui mis en oeuvre pour l'application comme traitement médical ou thérapeutique est un matériau composite de ruthénium à ligand nitrosyle irradié dans une gamme spectrale propre à libérer le ligand nitrosyle.

Dans le cas d'une réalisation par un procédé de type sol-gel, selon la première mise en oeuvre envisagée, il est fait expressément référence au document WO 2010/081977, déjà cité, qui décrit un tel procédé.
La méthode sol-gel comporte les étapes successives suivantes:
- Hydrolyse,
- Condensation ou, autrement désignée, polymérisation ou gélification,
- Séchage.

Dans le cas de seconde mise en oeuvre envisagée avec une matrice biocompatible, l'on réalise les étapes de dissolution de la matrice biocompatible, de dispersion du complexe photochrome, de disposition dans un contenant, de séchage où l'on sélectionne la température et la durée, pour que dans la matrice réalisée, selon la forme du contenant, le complexe photochrome de ruthénium à ligand nitrosyle, à l'état cristallin et sous forme de nanoparticules, soit inséré dans les nanopores de la matrice.

Comme indiqué, selon les deux mises en oeuvre envisagées, l'on sélectionne comme matrice, une matrice de silice ou une matrice biocompatible, en particulier d'amidon ou d'agar-agar.

En combinaison, et dans le cas d'une matrice de silice, l'on sélectionne comme complexe photochrome de ruthénium à ligand nitrosyle un complexe de formule :
o [Ru(NO)X(py-R)₄]Y₂, [Ru(NO)X(py-RR')₄]Y₂, [Ru(NO)X(py-RR'R")₄]Y₂ ou [Ru(NO)X(py)₄]Y₂ où :
   - py désigne la pyridine et Ru le ruthénium,
   - X est choisi dans la famille comprenant Cl, Br, OH,
   - Y est choisi dans la famille comprenant Cl, Br, BF₄, PF₆,
   - R, R', R" est choisi dans la famille comprenant l'hydrogène, les groupements alkyle, les groupements alcool, les groupements aldéhyde, les groupements cétone, les groupements ester, les groupements éther, les groupements amine, les groupements amide et les groupements halogénés,
o ou de formule [RuCl(NO)(py)₄](PF₆]₂.½H₂O.

Dans le cas d'une matrice biocompatible, l'on sélectionne comme complexe photochrome de ruthénium à ligand nitrosyle un complexe de formule [Ru(NO)X(py-R)₄]Y₂, [Ru(NO)X(py-RR')₄]Y₂, [Ru(NO)X(py-RR'R")₄]Y₂ ou [Ru(NO)X(py)₄]Y₂ où :
- py désigne la pyridine et Ru le ruthénium,
- X est choisi dans la famille comprenant Cl, Br, OH,
- Y est choisi dans la famille comprenant Cl, Br, BF₄,
- R, R', R" est choisi dans la famille comprenant l'hydrogène, les groupements alkyle, les groupements alcool, les groupements aldéhyde, les groupements cétone, les groupements ester, les groupements éther, les groupements amine, les groupements amide et les groupements halogénés.

Les groupements alkyle comprennent par exemple les alkyles ayant 1 à 4 atomes de carbone, linéaires ou ramifiés, tels que le méthyle, l'éthyle, le n-propyle, l'iso-propyle, ou le n-butyle.

Les groupements alcool comprennent par exemple -OH ou les chaînes alkyle ayant 1 à 4 atomes de carbones substitués par un ou plusieurs groupes -OH.

Les groupements aldéhyde comprennent par exemple -COH ou les chaînes alkyles ayant 1 à 4 atomes de carbones substitués par un ou plusieurs groupes -COH.

Les groupements ester comprennent par exemple -COR₁, où R₁ est un alkyle ayant 1 à 4 atomes de carbones.

Les groupements éther comprennent par exemple -OR₁, où R₁ est un alkyle ayant 1 à 4 atomes de carbones.

Les groupements amine comprennent par exemple -NH₂, -NHR, ou -NR₁R₂ où R₁ et R₂ sont indépendamment des alkyles ayant 1 à 4 atomes de carbones.

Les groupements amide comprennent par exemple -CONH₂, -CONHR₁ ou -CONR₁R₂ où R₁ et R₂ sont indépendamment des alkyles ayant 1 à 4 atomes de carbones.

Les groupements halogénés comprennent par exemple le chlore, le brome, l'iode ou le fluor ou les chaînes alkyles ayant 1 à 4 atomes de carbones substitués par un ou plusieurs atomes d'halogène, tels que le chlore, le brome, l'iode ou le fluor.

La pyridine py, la pyridine monosubstituée py-R, la pyridine bisubstituée py-RR' et la pyridine tri substituée py-RR'R" peuvent être illustrées par les structures schématiques suivantes :
Pyridine 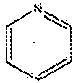
Pyridine monosubstituée py-R 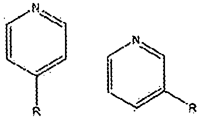
Pyridine bisubstituée py-RR' 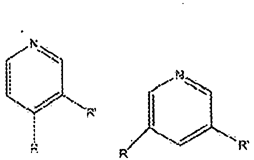
Pyridine tri substituée py-RR'R"

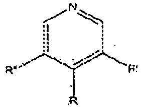

Dans une réalisation du procédé selon la première mise en oeuvre et le procédé sol-gel, l'on met en oeuvre, comme précurseur, le tétraméthoxyorthosilane - TMOS -. Dans d'autres réalisations possibles, l'on met en oeuvre le vinyltriéthoxysilane - VTES -, voire le tétraéthoxyorthosilane - TEOS -, ou plus généralement des alkoxydes M(OR)x, où M est un métal, tel que l'aluminium ou le titane, x un nombre entier pouvant aller jusqu'à 4, et R un groupement alkyle (ayant 1 à 4 atomes de carbone), fonctionnellement équivalents.

Comme indiqué, l'on a mis en évidence, de façon surprenante, qu'un matériau composite de ruthénium à ligand nitrosyle tel que précédemment décrit, une fois irradié dans une gamme spectrale propre à libérer le ligand nitrosyle pouvait avoir des applications pour un traitement médical ou thérapeutique fondé sur le rôle de NO.

Le traitement médical ou thérapeutique comprend par exemple :
- le traitement des maladies cardiovasculaires, tels que l'infarctus.
- le traitement de cancers, tels que les cancers de la peau.
- le traitement de maladies oculaires, telles que la dégénérescence maculaire.

Plus spécialement, le matériau composite de ruthénium à ligand nitrosyle est irradié dans une gamme spectrale propre à libérer le ligand nitrosyle de façon locale et photocontrôlée, par exemple dans la gamme spectrale de l'ultraviolet ou du visible, c'est-à- dire dans une gamme de longueur d'ondes comprise entre 300 nm et 700 nm.

Selon une autre mise en oeuvre possible, le matériau composite de ruthénium à ligand nitrosyle est irradié dans la gamme spectrale du proche infrarouge, c'est-à-dire dans une gamme de longueur d'ondes comprise entre 700 nm et 1100 nm.

Le traitement médical ou thérapeutique peut être mis en oeuvre par photothérapie dynamique. On peut ainsi soit administrer le matériau composite de ruthénium à ligand nitrosyle par voie orale, soit l'appliquer de façon percutanée, puis on irradie la zone à traiter avec un rayonnement approprié de façon à libérer localement le ligand nitrosyle.

D'autre part, selon une mise en oeuvre possible, le matériau composite de ruthénium à ligand nitrosyle employé pour l'application médicale ou thérapeutique considérée est en solution.

L'invention a également pour objet une composition pharmaceutique comprenant le matériau composite de ruthénium à ligand nitrosyle tel que définit précédemment et un excipient pharmaceutiquement acceptable. La composition pharmaceutique peut se présenter sous diverses formes, par exemple sous la forme d'un comprimé ou d'une gélule ou bien d'un patch.

La dose administrée dépendra bien entendu de l'application thérapeutique envisagée et du patient à traiter. La composition pharmaceutique administrée comprendra une quantité suffisante de matériau composite de ruthénium à ligand nitrosyle pour permettre de libérer localement une quantité thérapeutiquement efficace de NO. La quantité de NO libérée localement peut être contrôlée par la longueur d'onde et la durée de l'irradiation. On peut mesurer la quantité de NO libérée par exemple à l'aide d'une électrode NO, du test de Grieff ou bien du test Co TPP, et ajuster ainsi la longueur d'onde appropriée pour que le matériau libère NO.

## Revendications

1. Matériau composite de ruthénium à ligand nitrosyle propre à libérer le ligand nitrosyle lorsqu'il est irradié dans une gamme spectrale de l'ultraviolet ou du visible, pour son utilisation dans un traitement médical ou thérapeutique, ledit matériau comprenant un complexe photochrome de ruthénium à ligand nitrosyle de formule :
o [Ru(NO)X(py-R)₄]Y₂, [Ru(NO)X(py-RR')₄]Y₂, [Ru(NO)X(py-RR'R")₄]Y₂ ou [Ru(NO)X(py)₄]Y₂ où :
- py désigne la pyridine et Ru le ruthénium,
- X est choisi dans la famille comprenant Cl, Br, OH,
- Y est choisi dans la famille comprenant Cl, Br, BF₄, PF₆,
- R, R', R" est choisi dans la famille comprenant l'hydrogène, les groupements alkyle, les groupements alcool, les groupements aldéhyde, les groupements cétone, les groupements ester, les groupements éther, les groupements amine, les groupements amide et les groupements halogénés,
o ou de formule [RuCl(NO)(py)4](PF6]2.½H2O.

2. Matériau composite de ruthénium à ligand nitrosyle propre à libérer le ligand nitrosyle lorsqu'il est irradié dans une gamme spectrale du proche infrarouge, pour son utilisation dans un traitement médical ou thérapeutique, ledit matériau comprenant un complexe photochrome de ruthénium à ligand nitrosyle de formule :
o [Ru(NO)X(py-R)₄]Y₂, [Ru(NO)X(py-RR')₄]Y₂, [Ru(NO)X(py-RR'R")₄]Y₂ ou [Ru(NO)X(py)₄]Y₂ où :
- py désigne la pyridine et Ru le ruthénium,
- X est choisi dans la famille comprenant Cl, Br, OH,
- Y est choisi dans la famille comprenant Cl, Br, BF₄, PF₆,
- R, R', R" est choisi dans la famille comprenant l'hydrogène, les groupements alkyle, les groupements alcool, les groupements aldéhyde, les groupements cétone, les groupements ester, les groupements éther, les groupements amine, les groupements amide et les groupements halogénés,
o ou de formule [RuCL(NO)(py)4](PF6]2.½H2O.

3. Matériau composite de ruthénium à ligand nitrosyle pour son utilisation dans un traitement médical ou thérapeutique selon la revendication 1 ou 2, propre à être irradié dans une gamme spectrale de l'ultraviolet ou du visible ou du proche infrarouge et à libérer le ligand nitrosyle de façon locale et photocontrôlée.

4. Matériau composite de ruthénium à ligand nitrosyle, pour son utilisation dans un traitement médical ou thérapeutique selon l'une quelconque des revendications 1 à 3, réalisé par un procédé sol-gel avec :
▪ comme précurseur un alkoxysilane,
▪ une matrice de silice, et
▪ un complexe photochrome de ruthénium à ligand nitrosyle un complexe de formule :
o [Ru(NO)X(py-R)₄]Y₂, [Ru(NO)X(py-RR')₄]Y₂[Ru(NO)X(py-RR'R")₄]Y₂ ou [Ru(NO)X(py)₄]Y₂ où :
- py désigne la pyridine et Ru le ruthénium,
- X est choisi dans la famille comprenant Cl, Br, OH,
- Y est choisi dans la famille comprenant Cl, Br, BF₄, PF₆,
- R, R', R" est choisi dans la famille comprenant l'hydrogène, les groupements alkyle, les groupements alcool, les groupements aldéhyde, les groupements cétone, les groupements ester, les groupements éther, les groupements amine, les groupements amide et les groupements halogénés,
o ou de formule [RuCl(NO)(py)₄][PF₆]₂.½H2O.

5. Matériau composite de ruthénium à ligand nitrosyle, pour son utilisation dans un traitement médical ou thérapeutique selon l'une quelconque des revendications 1 à 4, dans laquelle l'alkoxysilane est sélectionnée dans le groupe comprenant le tétraméthoxyorthosilane - TMOS -, le vinyltriéthoxysilane - VTES -, le tétraéthoxyorthosilane - TEOS -, ou des alkoxydes M(OR)x, où M est un métal et R un groupement alkyle.

6. Matériau composite de ruthénium à ligand nitrosyle, pour son utilisation dans un traitement médical ou thérapeutique selon l'une quelconque des revendications 1 à 3 réalisé avec une matrice biocompatible, en particulier d'amidon ou d'agar-agar, et comme complexe photochrome de ruthénium à ligand nitrosyle un complexe de formule :
o [Ru(NO)X(py-R)₄]Y₂, [Ru(NO)X(py-RR')₄]Y₂, [Ru(NO)X(py-RR'R")₄]Y₂ ou [Ru(NO)X(py)₄]Y₂ où :
- py désigne la pyridine et Ru le ruthénium,
- X est choisi dans la famille comprenant Cl, Br, OH,
- Y est choisi dans la famille comprenant Cl, Br, BF₄,
- R, R', R" est choisi dans la famille comprenant l'hydrogène, les groupements alkyle, les groupements alcool, les groupements aldéhyde, les groupements cétone, les groupements ester, les groupements éther, les groupements amine, les groupements amide et les groupements halogénés.

7. Composition pharmaceutique comprenant le matériau composite de ruthénium à ligand nitrosyle tel que défini selon l'une quelconque des revendications 1, 4 à 6 et un excipient pharmaceutiquement acceptable.

8. Composition pharmaceutique selon la revendication 7, **caractérisée en ce qu'**elle se présente sous forme de comprimé, gélule ou patch.

## Patentansprüche

1. Ruthenium-Kompositmaterial mit einem Nitrosyl-Liganden, dazu geeignet, den Nitrosyl-Liganden freizusetzen, wenn es in einem ultravioletten oder sichtbaren Spektralbereich bestrahlt wird, für die Verwendung bei einer medizinischen oder therapeutischen Behandlung, wobei das Material einen photochromen Komplex von Ruthenium mit einem Nitrosyl-Liganden der folgenden Formel:
∘ [Ru(NO)X(py-R)₄]Y₂, [Ru(NO)X(py-RR')₄]Y₂, [Ru(NO)X(py-RR'R")₄]Y₂ oder [Ru(NO)X(py)₄]Y₂, wobei:
- py für Pyridin steht und Ru für Ruthenium steht,
- X aus der Familie ausgewählt ist, die Cl, Br, OH umfasst,
- Y aus der Familie ausgewählt ist, die Cl, Br, BF₄, PF₆ umfasst,
- R, R', R" aus der Familie ausgewählt ist, die Wasserstoff, Alkylgruppen, Alkoholgruppen, Aldehydgruppen, Ketongruppen, Estergruppen, Ethergruppen, Amingruppen, Amidgruppen und halogenierte Gruppen umfasst,
∘ oder der Formel [RuCl (NO) (py)4] (PF6]2.½H₂O umfasst.

2. Ruthenium-Kompositmaterial mit einem Nitrosyl-Liganden, dazu geeignet, den Nitrosyl-Liganden freizusetzen, wenn es in einem Nah-Infrarot-Spektralbereich bestrahlt wird, für die Verwendung bei einer medizinischen oder therapeutischen Behandlung, wobei das Material einen photochromen Komplex von Ruthenium mit einem Nitrosyl-Liganden der folgenden Formel:
∘ [Ru(NO)X(py-R)₄]Y₂, [Ru(NO)X(py-RR')₄]Y₂, [Ru(NO)X(py-RR'R")₄]Y₂ oder [Ru(NO)X(py)₄]Y₂, wobei:
- py für Pyridin steht und Ru für Ruthenium steht,
- X aus der Familie ausgewählt ist, die Cl, Br, OH umfasst,
- Y aus der Familie ausgewählt ist, die Cl, Br, BF₄, PF₆ umfasst,
- R, R', R" aus der Familie ausgewählt ist, die Wasserstoff, Alkylgruppen, Alkoholgruppen, Aldehydgruppen, Ketongruppen, Estergruppen, Ethergruppen, Amingruppen, Amidgruppen und halogenierte Gruppen umfasst,
∘ oder der Formel [RuCl(NO)(py)4](PF6]2.½H₂O umfasst.

3. Ruthenium-Kompositmaterial mit einem Nitrosyl-Liganden für die Verwendung bei einer medizinischen oder therapeutischen Behandlung nach Anspruch 1 oder 2, dazu geeignet, in einem ultravioletten oder sichtbaren oder NahInfrarot-Spektralbereich bestrahlt zu werden und den Nitrosyl-Liganden lokal und auf lichtgesteuerte Weise freizusetzen.

4. Ruthenium-Kompositmaterial mit einem Nitrosyl-Liganden für die Verwendung bei einer medizinischen oder therapeutischen Behandlung nach einem der Ansprüche 1 bis 3, hergestellt durch ein Sol-Gel-Verfahren mit:
▪ einem Alkoxysiloxan als Vorstufe,
▪ einer Siliziumdioxid-Matrix und
▪ einem photochromen Komplex von Ruthenium mit einem Nitrosyl-Liganden, einem Komplex der folgenden Formel:
∘ [Ru(NO)X(py-R)₄]Y₂, [Ru(NO)X(py-RR')₄]Y₂, [Ru(NO)X(py-RR'R")₄]Y₂ oder [Ru(NO)X(py)₄]Y₂, wobei:
- py für Pyridin steht und Ru für Ruthenium steht,
- X aus der Familie ausgewählt ist, die Cl, Br, OH umfasst,
- Y aus der Familie ausgewählt ist, die Cl, Br, BF₄, PF₆ umfasst,
- R, R', R" aus der Familie ausgewählt ist, die Wasserstoff, Alkylgruppen, Alkoholgruppen, Aldehydgruppen, Ketongruppen, Estergruppen, Ethergruppen, Amingruppen, Amidgruppen und halogenierte Gruppen umfasst,
∘ oder der Formel [RuCl(NO)(py)₄](PF₆]₂.½H₂O umfasst.

5. Ruthenium-Kompositmaterial mit einem Nitrosyl-Liganden für die Verwendung bei einer medizinischen oder therapeutischen Behandlung nach einem der Ansprüche 1 bis 4, wobei das Alkoxysilan aus der Gruppe ausgewählt ist, die Tetramethoxyorthosilan - TMOS -, Vinyltriethoxysilan - VTES -, Tetraethoxyorthosilan - TEOS - oder Alkoxide M(OR)x umfasst, wobei M ein Metall ist und R eine Alkylgruppe ist.

6. Ruthenium-Kompositmaterial mit einem Nitrosyl-Liganden für die Verwendung bei einer medizinischen oder therapeutischen Behandlung nach einem der Ansprüche 1 bis 3, hergestellt mit einer biokompatiblen Matrix, insbesondere aus Stärke oder Agar-Agar, und als photochromen Komplex aus Ruthenium mit einem Nitrosyl-Liganden einem Komplex der folgenden Formel:
∘ [Ru(NO)X(py-R)₄]Y₂, [Ru(NO)X(py-RR')₄]Y₂, [Ru(NO)X(py-RR'R")₄]Y₂ oder [Ru(NO)X(py)₄]Y₂, wobei:
- py für Pyridin steht und Ru für Ruthenium steht,
- X aus der Familie ausgewählt ist, die Cl, Br, OH umfasst,
- Y aus der Familie ausgewählt ist, die Cl, Br, BF₄, PF₆ umfasst,
- R, R', R" aus der Familie ausgewählt ist, die Wasserstoff, Alkylgruppen, Alkoholgruppen, Aldehydgruppen, Ketongruppen, Estergruppen, Ethergruppen, Amingruppen, Amidgruppen und halogenierte Gruppen umfasst.

7. Pharmazeutische Zusammensetzung, umfassend das Ruthenium-Kompositmaterial mit einem Nitrosyl-Liganden nach einem der Ansprüche 1, 4 und 6 und einen pharmazeutisch annehmbaren Excipienten.

8. Pharmazeutische Zusammensetzung nach Anspruch 7, **dadurch gekennzeichnet, dass** sie in Form eine Tablette, einer Kapsel oder eines Pflasters dargereicht wird.

## Claims

1. A composite material of ruthenium with a nitrosyl ligand able to release the nitrosyl ligand when it is irradiated in an ultraviolet or visible spectral range, for use in a medical or therapeutic treatment, said material comprising a photochromic complex of ruthenium with a nitrosyl ligand, of formula:
o [Ru(NO)X(py-R)₄]Y₂, [Ru(NO)X(py-RR')₄]Y₂, [Ru(NO)X(py-RR'R")₄]Y₂ or [Ru(NO)X(py)₄]Y₂
wherein:
- py denotes pyridine and Ru denotes ruthenium,
- X is chosen from the family comprising Cl, Br and OH,
- Y is chosen from the family comprising Cl, Br, BF₄ and PF₆,
- R, R', R" are chosen from the family comprising hydrogen, alkyl groups, alcohol groups, aldehyde groups, ketone groups, ester groups, ether groups, amine groups, amide groups and halogenated groups,
o or of formula [RuCl(NO)(py)₄](PF₆)₂.½H₂O.

2. A composite material of ruthenium with a nitrosyl ligand able to release the nitrosyl ligand when it is irradiated in a near-infrared spectral range, for use in a medical or therapeutic treatment, said material comprising a photochromic complex of ruthenium with a nitrosyl ligand, of formula:
o [Ru(NO)X(py-R)₄]Y₂, [Ru(NO)X(py-RR')₄]Y₂, [Ru(NO)X(py-RR'R")₄]Y₂ or [Ru(NO)X(py)₄]Y₂
wherein:
- py denotes pyridine and Ru denotes ruthenium,
- X is chosen from the family comprising Cl, Br and OH,
- Y is chosen from the family comprising Cl, Br, BF₄ and PF₆,
- R, R', R" are chosen from the family comprising hydrogen, alkyl groups, alcohol groups, aldehyde groups, ketone groups, ester groups, ether groups, amine groups, amide groups and halogenated groups,
o or of formula [RuCl(NO)(py)₄](PF₆)₂.½H₂O.

3. The composite material of ruthenium with a nitrosyl ligand for use in a medical or therapeutic treatment as claimed in claim 1 or 2, able to be irradiated in an ultraviolet or visible or near-infrared spectral range and to release the nitrosyl ligand locally in a photocontrolled manner.

4. The composite material of ruthenium with a nitrosyl ligand for use in a medical or therapeutic treatment as claimed in any one of claims 1 to 3, prepared by means of a sol-gel process with:
▪ an alkoxysilane as precursor,
▪ a silica matrix, and
▪ a photochromic complex of ruthenium with a nitrosyl ligand, a complex of formula:
o [Ru(NO)X(py-R)₄]Y₂, [Ru(NO)X(py-RR')₄]Y₂[Ru(NO)X(py-RR'R")₄]Y₂ or [Ru(NO)X(py)₄]Y₂
wherein:
- py denotes pyridine and Ru denotes ruthenium,
- X is chosen from the family comprising Cl, Br and OH,
- Y is chosen from the family comprising Cl, Br, BF₄ and PF₆,
- R, R', R" are chosen from the family comprising hydrogen, alkyl groups, alcohol groups, aldehyde groups, ketone groups, ester groups, ether groups, amine groups, amide groups and halogenated groups,
o or of formula [RuCI(NO)(py)₄](PF₆)₂.½H₂O.

5. The composite material of ruthenium with a nitrosyl ligand for use in a medical or therapeutic treatment as claimed in any one of claims 1 to 4, wherein the alkoxysilane is selected from the group comprising tetramethoxyorthosilane - TMOS -, vinyltriethoxysilane - VTES -, tetraethoxyorthosilane - TEOS -, or alkoxides M(OR)x, wherein M is a metal and R is an alkyl group.

6. The composite material of ruthenium with a nitrosyl ligand for use in a medical or therapeutic treatment as claimed in any one of claims 1 to 3, prepared with a biocompatible matrix, in particular of starch or of agar-agar, and, as photochromic complex of ruthenium with a nitrosyl ligand, a complex of formula:
o [Ru(NO)X(py-R)₄]Y₂, [Ru(NO)X(py-RR')₄]Y₂, [Ru(NO)X(py-RR'R")₄]Y₂ or [Ru(NO)X(py)₄]Y₂
wherein:
- py denotes pyridine and Ru denotes ruthenium,
- X is chosen from the family comprising Cl, Br and OH,
- Y is chosen from the family comprising Cl, Br and BF₄,
- R, R', R" are chosen from the family comprising hydrogen, alkyl groups, alcohol groups, aldehyde groups, ketone groups, ester groups, ether groups, amine groups, amide groups and halogenated groups.

7. A pharmaceutical composition comprising the composite material of ruthenium with a nitrosyl ligand as defined in any one of claims 1 and 4 to 6, and a pharmaceutically acceptable excipient.

8. The pharmaceutical composition as claimed in claim 7, **characterized in that** it is in the form of a tablet, a gel capsule or a patch.
